# EUROPÄISCHE PATENTSCHRIFT

(11) **EP 1 112 496 B1**
(45) Veröffentlichungstag und Bekanntmachung des Hinweises auf die Patenterteilung: **05.07.2006**
(21) Anmeldenummer: 99968715.5
(22) Anmeldetag: 03.09.1999
(51) Int. Cl.: G01N 33/573, C07K 16/40, C12N 9/66

(54) **DIAGNOSTISCHES VERFAHREN ZUR ERKENNUNG EINER PANKREASFUNKTIONSSTÖRUNG**
DIAGNOSTIC METHOD FOR DETECTING DISTURBANCES OF THE PANCREAS
METHODE DIAGNOSTIQUE PERMETTANT DE DETECTER UNE PERTURBATION DE LA FONCTION PANCREATIQUE

(30) Priorität: 08.09.1998 DE 19840900; 25.05.1999 DE 19923892
(43) Veröffentlichungstag der Anmeldung: 04.07.2001
(73) Patentinhaber: Bioserv Analytik und Medizinprodukte GmbH, 18059 Rostock (DE)
(72) Erfinder: HEINRICH, Hans-Werner, D-17498 Riemserort (DE); KLEINERT, Rainer, D-17493 Greifswald (DE); MEYER, Udo, D-18239 Hastorf (DE); WAGNER, Heinz-Jürgen, D-13125 Berlin (DE)
(74) Vertreter: Baumbach, Friedrich
(86) Internationale Anmeldenummer: PCT/DE1999/002816
(87) Internationale Veröffentlichungsnummer: WO 2000/014542

(56) Entgegenhaltungen:
- US-A- 5 622 837

## Beschreibung

Die Erfindung betrifft ein diagnostisches Verfahren zur Erkennung einer Pankreasfunktionsstörung. Anwendungsgebiete sind die Medizin und die pharmazeutische Industrie.

Funktionsstörungen der Bauchspeicheldrüse können das Ergebnis unterschiedlicher Erkrankungen sein, deren Diagnose unter anderem durch die Bestimmung funktioneller Kriterien untermauert werden sollte. Die häufigsten Erkrankungen des Pankreas sind die chronisch rezidivierende und die akute Pankreatitis.

Die chronische Pankreatitis ist eine schleichende progrediente Erkrankung, bei der das funktionstüchtige Pankreasgewebe im Rahmen eines sklerotisierenden Prozesses allmählich degeneriert. Sie ist charakterisiert durch ihr klinisches Beschwerdebild (abdominelle Schmerzen, Steatorrhoe, Gewichtsverlust), typische morphologische Veränderungen der Drüse (Verkalkungen, dilatierter, unregelmäßig begrenzter Ductus pancreaticus) sowie einen progredienten exokrinen und endokrinen Funktionsverlust (Maldigestion, Diabetes mellitus). Die chronische Pankreatitis hat eine Inzidenz von 6 bis 8 Neuerkrankungen pro 100 000 Einwohnern pro Jahr in Westeuropa. Die Diagnose der chronischen Pankreatitis wird wegen steigenden Alkoholkonsums immer häufiger gestellt.

Zur Untermauerung klinischer und morphologischer Befunde können verschiedene funktionelle Kriterien bestimmt werden. Die sensitivste Analysenmethode stellt der Sekretin-Caerulin- bzw. Sekretin-Cholezystokinin-Test dar, der jedoch den Patienten stark belastet und einen hohen Zeitaufwand erfordert. Als indirekte Testmethoden werden Lundh-, NBT-PABA- und Pancreoauryl-Test angewendet. Auch die Bestimmung von Trypsin im Serum oder Chymotrypsin im Stuhl haben eine gewisse praktische Bedeutung. Alle diese indirekten Testmethoden haben den Nachteil einer ungenügenden Spezifität.

Die Bestimmung der Elastase 1 ist von allen verfügbaren indirekten Funktionstesten der Test mit der höchsten Sensitivität und Spezifität (Löser,C., Therapie & Erfolg 1997; 1:411-413). Er hat sich in den letzten Jahren für die tägliche Praxis als Standard für die exokrinen Pankreasfunktionsdiagnostik durchgesetzt. Grundlage für diesen Test sind polyklonale Antikörpern gegen Elastase 1 (Elastase 1-RIA, Abbott) bzw. mono- und/oder polyklonalen Anti-Elastase-1-Antikörpern, die durch Immunisierung mit einem Antigen, das die Aminosäuresequenz Thr-Met-Val-Ala-Gly-Gly Asp-Ile-Arg oder immunologisch wirksame Teilpeptide davon enthält, gewonnen werden (EP 0 547 059 B1).

Pankreas-Elastase ist ein proteolytisches Verdauungsenzym. Im Vergleich zu den gebräuchlichen Parametern der Pankreasdiagnostik (z.B. Chymotrypsinaktivität im Stuhl) hat die quantitative Elastase-Bestimmung entscheidende Vorteile. Das Enzym wird ausschließlich im Pankreas gebildet und weist eine außergewöhnliche Stabilität während der Darmpassage auf, d.h., die Konzentration der Elastase spiegelt die Sekretionsleistung des Pankreas wieder.

Trotz Überlegenheit gegenüber anderen exokrinen Parametern wird mit dem herkömmlichen Elastase 1-ELISA in zu vielen Fällen Pankreas-Elastase nicht nachgewiesen, obwohl sie in beträchtlichen Konzentrationen vorhanden ist.

Fujimoto und Mitarbeiter (Fujimoto, K., Clinical Biochemistry 1983; 16 (1) :26-27) offenbaren ein Verfahren in dem der Gehalt der Eiastasen 1 und 2 im Serum von Patienten mit Pankreatitis bestimmt wird.

Ziel der Erfindung ist daher die Entwicklung eines sensitiveren Verfahrens zur Erkennung einer Pankreasfunktionsstörung auf der Grundlage des Nachweises pankreatischer Elastase.

Durch systematische Untersuchung von Stuhlproben, die mit dem herkömmlichen ELISA nicht auf Elastase 1 reagierten, konnten nach elektrophoretischer Trennung Proben hergestellt werden, die Elastase enthielten. Die weitere Charakterisierung ergab, daß es sich bei diese Proteinen um verschiedene Isoenzyme handelt, die offensichtlich mit den kommerziellen Testantikörpern nicht erkannt werden. Es gibt offensichtlich für dieses Enzym einen ausgeprägten genetischen Polymorphismus, der sich in der Fachliteratur auch in der Beschreibung von Pankreas-Elastase 1, 2 und 3 widerspiegelt. Durch eigene Untersuchungen konnte auch nachgewiesen werden, daß zumindest 2 Isoenzyme gleichzeitig vorkommen können. Es konnte auch gezeigt werden, daß sich diese Elastasen in ihrer Konzentrationsabhängigkeit zur Pankreasschädigung genauso verhalten, wie die Elastase 1.

Im Gegensatz zu den bekannten Lösungen, die sich auf den spezifischen Nachweis der Pankreas-Elastase 1 beschränken, wurde überraschenderweise gefunden, daß durch den Nachweis möglichst jedoch alle Pankreas-Elastase-Isoformen, bekannt sind derzeit die Pankreas-Elastasen 1, 2 und 3, die Aussagefähigkeit im Bezug auf die Pankraesfunktion entscheidend verbessert werden kann. Die Erfindung betrifft daher ein Verfahren zur Herstellung von anti-Elastase-Antikörpern auf übliche Weise, das jedoch dadurch gekennzeichnet ist, daß die verwendeten spezifischen Antigene einzeln oder in Kombination alle bekannten Elastase-Isoenzyme oder Teilstücke von diesen oder kreuzreagierende synthetische Sequenzen repräsentieren.

Teilstücke werden vorzugsweise durch Peptidsynthese gewonnen, wobei die Aminosäuresequenz vorher mittels strukturanalytischer Methoden aus der Gesamtsequenz abgeleitet oder nach Proteinsequenzierung ermittelt wird. Wenngleich die Peptide bereits allein eine Antikörperinduktion auslösen, hat es sich erfindungsgemäß als zweckmäßig erwiesen, diese Peptide an übliche Carriersubstanzen wie Hämocyanin zu binden. Mit den erfindungsgemäßen Peptiden ist es möglich, monoklonale wie polyklonale Antikörper zu erzeugen.

Für die Herstellung der erfindungsgemäßen polyklonalen Antipeptidantikörper werden Versuchstiere wie Kaninchen, Meerschweinchen, Ziegen, Hühner oder Fische in bekannter Weise mit den Peptiden immunisiert. Für die Herstellung monoklonaler Antikörper werden die Peptide in bekannter Weise für die Induktion spezifischer B-Zellen verwendet, die nach Fusionierung mit Myelomzellen Hybridomzellen generieren, die nach bekannten Klonierungsverfahren in Zellinien kultiviert werden, die spezifische monoklonale Antikörper sezernieren. Die erfindungsgemäßen mono- oder polyklonalen Antikörper reagieren nur mit dem verwendeten spezifischen Epitopen bzw. mit allen bekannten Elastase-Isoenzymen.
Es konnte nachgewiesen werden, daß Antikörper gegen die Peptide A-V-K-E-G-P-E-Q-V-I-P-I-N, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-I, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-K-G-N-D, G-P-L-N-C-Q-AS-D-G-R-W, G-A-L-P-D-D-L-K-Q-G-R-L, S-L-Q-Y-E-K-S-G-S-F-Y, F-G-C-N-T-R-R-K-P-T-V-F-T, hochspezifisch mit den Isoformen der Pankreas-Elastase reagieren und keine unspezifische Reaktion mit anderen Stuhlbestandteilen eingehen.
Ein weiterer Gegenstand der Erfindung ist die Verwendung der erfindungsgemäßen Elastase-Antikörper für den Nachweis und die Quantifizierung aller bekannter Elastase-Isoenzyme in Körperflüssigkeiten und Stuhl. Die Erfindung betrifft daher auch Nachweissysteme, vorzugsweise ein immunchemisches Nachweissystem zur Feststellung der Funktionalität des Pankreas als Hilfsmittel zur Erkennung von Funktionsstörungen dieses Organs. Dazu können die spezifischen Antikörper an jeden geeigneten Träger adsorptiv oder chemisch mit bekannten Kopplungsverfahren gebunden werden. Als Träger eignen sich Membranen oder Partikel. Mit einem erfindungsgemäßen Sandwich-ELISA unter Verwendung von kreuzreaktiven oder einer Kombination unterschiedlicher Epitopantikörper lassen sich schnell und spezifisch Pankreas-Elastase in Stuhl und Serum bzw. Plasma nachweisen und quantifizieren.

Er dient zur Diagnose bzw. dem Ausschluß einer Pankreasbeteiligung bei Abdominalbeschwerden und exokriner Pankreasinsuffiziens.

Es gibt auch Fälle, in denen bereits die Erfassung von Elastase 1 genügt, um eine sichere Diagnose von Pankreasstörungen stellen zu können. In diesen Fällen wird das erfindungsgemäße Verfahren folgendermaßen geführt:

Die DNA-Sequenz für humane Elastase 1 (JP 1987000276-A/6) wurde in die Aminosäuresequenz übertragen. Unter Verwendung üblicher Proteinstrukturprogramme konnten mehrere Aminosäuresequenzen identifiziert werden, die eine potentielle Epitopstruktur aufweisen. Es konnte nachgewiesen werden, daß Antikörper gegen die Peptide A-V-K-E-G-P-E-Q-V-I-P-I-N, Y-T-N-G-P-L-P-D-K-L-Q-Q-A-R, R-S-G-C-N-G-D-S-G-G-P-L-N und G-P-L-N-C-P-T-E-D-G-G-W-Q hochspezifisch Elastase 1 binden und keine unspezifische Reaktion mit anderen Stuhlbestandteilen eingehen.

Die Erfindung betrifft ein Verfahren zur Herstellung von anti-Elastase-Antikörpern auf übliche Weise, das jedoch dadurch gekennzeichnet ist, daß die verwendeten spezifischen Antigene zuvor mittels strukturanalytischer Methoden aus der Aminosäuresequenz abgeleitet und chemisch synthetisiert wurden. Erfindungsgemäß ist es auch möglich, Teile dieser synthetischen Peptide für die Herstellung von Antikörpern zu verwenden. Wenngleich die Peptide allein eine Antikörperinduktion auslösen, hat es sich erfindungsgemäß als zweckmäßig erwiesen, diese Peptide an übliche Carriersubstanzen wie Hämocyanin zu binden. Mit den erfindungsgemäßen Peptiden ist es möglich, sowohl monoklonale wie polyklonale Antikörper zu erzeugen.
Für die Herstellung der erfindungsgemäßen polyklonalen Antipeptidantikörper werden Versuchstiere wie Kaninchen, Meerschweinchen, Ziegen, Hühner oder Fische in bekannter Weise mit den Peptiden immunisiert. Für die Herstellung monoklonaler Antikörper werden die Peptide in bekannter Weise für die Induktion spezifischer B-Zellen verwendet, die nach Fusionierung mit Myelomzellen Hybridomzellen generieren, die nach bekannten Klonierungsverfahren in Zellinien kultiviert werden, die spezifische monoklonale Antikörper sezernieren. Die erfindungsgemäßen mono- oder polyklonalen Antikörper reagieren nur mit dem verwendeten spezifischen Epitop bzw. der reifen Elastase 1.

Ein weiterer Gegenstand der Erfindung ist die Verwendung der erfindungsgemäßen Elastase 1-epitopspezifischen Antikörper für den Nachweis und die Quantifizierung von Elastase 1 in Körperflüssigkeiten und Stuhl. Die Erfindung betrifft daher auch ein immunchemisches Nachweissystem zur Feststellung der Funktionalität des Pankreas als Hilfsmittel zur Erkennung von Funktionsstörungen dieses Organs. Dazu können die spezifischen Antikörper an jeden geeigneten Träger adsorptiv oder chemisch mit bekannten Kopplungsverfahren gebunden werden. Als Träger eignen sich Membranen oder Partikel. Mit einem erfindungsgemäßen Sandwich-ELISA unter Verwendung von je zwei unterschiedlichen Epitopantikörpern läßt sich schnell und spezifisch Elastase 1 in Stuhl und Serum bzw. Plasma nachweisen und quantifizieren.

### Ausführungsbeispiel 1 - Herstellung spezifischer anti-Peptid-Antikörper, die gegen definierte Abschnitte der reifen humanen Elastase 1 gerichtet sind.

Mittels Festphasensynthese nach Merrifield werden die Peptide mit den Aminosäuresequenzen NH₂-A-V-K-E-G-P-E-Q-V-I-P-I-N-COOH, NH₂-Y-T-N-G-P-L-P-D-K-L-Q-Q-A-R-COOH, NH₂-R-S-G-C-N-G-D-S-G-G-P-L-N-COOH und NH₂-G-P-L-N-C-P-T-E-D-G-G-W-Q-COOH synthetisiert. Die Peptide werden mit bekannten Verfahren an Napfschneckenhämocyanin (KLH) gekoppelt (1 mg Peptid/mg KLH). Je 300 µg dieses Konjugates werden unter Zusatz von Freundschen Adjuvants für die Immunisierung von Kaninchen bzw. Huhn verwendet. Nach 3maliger Vakzination werden die Tiere entblutet. Nach Gewinnung des Serums wird die Spezifität der Antiseren in einem ELISA getestet. Dazu wird freies Peptid an die Oberfläche der Kavitäten von Mikrotiterplatten adsorbiert. Nach Inkubation der Kavitäten mit den Antiseren werden diese gründlich gewaschen. Unter Verwendung von Antikaninchen- bzw. Antihuhn-POD-Konjugat und TMB als Substrat werden in üblicher Weise die Antigen-Antikörperreaktion detektiert. Jedes Antiserum reagiert nur mit dem homologen Peptid.

### Ausführungsbeispiel 2 - Nachweis der Spezifität der erfindungsgemäßen Antikörper

Die Elastase 1 - Spezifität kann im Westernblot nachgewiesen werden. Dazu werden grob- bzw. hochgereinigte Elastase 1 aus Stuhl mittels Polyacrylamidgel-Elektropherese entsprechend ihrer relativen Molmasse von begleitenden Verunreinigungen getrennt. Die Proteinzonen aus dem Gel werden mit Hilfe einer 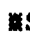 Semidry-blotting"-Apparatur auf Nitrozellulose übertragen. Nach Absättigung der freien Bindungsstellen der Membran mit resuspendierter Trockenmagermilch werden die Membranen mit den 1 : 500 verdünnten anti-Peptid-Antiseren inkubiert. Nach intensiven Waschen der Membranen zur Entfernung aller unspezifisch gebundenen Antikörper werden die Membranen mit Phosphatase - markierten anti-Kaninchen-Antikörpern inkubiert.

Die spezifisch gebundenen sekundären Antikörper, die nach Waschen auf der Membran verblieben, werden nach Zugabe des Substrates sichtbar gemacht. Dabei zeigt sich, daß in den verwendeten Proben ausschließlich Elastase nachgewiesen wurde.

### Ausführungsbeispiel 3 - Bestimmung der Elastase 1 in Stuhl unter Verwendung der erfindungsgemäßen Antikörper in einem ELISA

Die Elatase 1 in Serumproben oder in Stuhlproben wird in einem Festphasen-Enzymimmunoassay, der auf der Sandwichtechnik basiert, bestimmt. Ein polyklonaler Antikörper, der gegen Epitope der Elastase 1 gerichtet ist, wird in einem Karbonat/Bikarbonat-Puffergemisch, pH 9,6 gelöst und in die Wells einer Mikrotiterplatte gegeben. Nach der Inkubation bei 4 °C über 12 h werden die nicht gebundenen Antikörper durch Waschen mit PBS entfernt. Die noch freien Bindungsstellen des Trägermaterials werden durch einen PBS-Puffer, der Ethanol-amin und Tween 20 enthält, geblockt. Das Blocken findet bei Raumtemperatur über 90 min statt. Nach dem Waschen werden die in PBS verdünnten Serum- bzw. Stuhlproben in die Wells pipettiert. Die 60-minütige Inkubation bei Raumtemperatur wird durch Waschen beendet. Ein zweiter Elastase 1 spezifischer polyklonaler Antikörper, der mit Biotin konjugiert ist, wird zu der an den ersten Antikörper gebundenen Elastase gegeben.

Nach einer Inkubation von 30 Minuten und dem Waschprozeß wird der biotinmarkierte Antikörper mit Peroxidase-konjugiertem Streptavidin nachgewiesen. Durch den letzten Waschschritt erfolgt die Beseitigung des nicht gebunden Streptavidins. Anschließend wird TMB als Substrat für die Peroxidase dazugegeben und nach einer definierten Zeit wird die Farbreaktion durch Zugabe von HCl abgestoppt. Gemessen wird die Änderung der optischen Dichte. Die Intensität der Farbreaktion ist proportional der Elastase 1-Konzentration der Probe.

### Ausführungsbeispiel 4 - Herstellung spezifischer anti-Peptid-Antikörper, die gegen definierte Abschnitte von Isoformen der Pankreas-Elastase gerichtet sind.

Mittels Festphasensynthese nach Merrifield werden die Peptide mit den Aminosäuresequenzen A-V-K-E-G-P-E-Q-V-I-P-I-N, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-I, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-K-G-N-D, G-P-L-N-C-Q-A-S-D-G-R-W, G-A-L-P-D-D-L-K-Q-G-R-L, S-L-Q-Y-E-K-S-G-S-F-Y, F-G-C-N-T-R-R-K-P-T-V-F-T synthetisiert. Die Peptide werden mit bekannten Verfahren an Napfschneckenhämocyanin (KLH) gekoppelt (1 mg Peptid/mg KLH). Je 300 µl dieses Konjugates werden unter Zusatz von Freundschen Adjuvants für die Immunisierung von Kaninchen oder Huhn verwendet. nach dreimaliger Vakzination werden die Tiere entblutet. Nach Gewinnung der Antikörper (Reinigung über Protein A-Säule bzw. fraktionierte Fällung) wird ihre Spezifität in einem ELISA getestet. Dazu wird freies Peptid an die Oberfläche der Kavitäten von Mikrotiterplatten adsorbiert. Nach Inkubation der Kavitäten mit den homologen bzw. heterologen Antikörpern werden diese gründlich gewaschen. Unter Verwendung von Anti-Kaninchen- bzw. Anti-Huhn-POD-Konjugaten und TMB als Substrat werden in üblicher Weise die Antigen-Antikörper-Reaktionen detektiert. Jeder Antikörper reagiert nur mit dem homologen Peptid.

### Ausführungbeispiel 5 - Nachweis der Spezifität der erfindungsgemäßen Antikörper

Die Spezifität der Antikörper für verschiedene Isoformen der Pankreas-Elastase kann im Western-Blot nachgewiesen werden. Dazu werden grob- und hochgereinigte Elastaseproben aus Stuhl und Pankreassaft mittels Polyacrylamid-Gelelektrophorese entsprechend ihrer relativen Molmasse von begleitenden Verunreinigungen getrennt. Die Proteinzonen aus dem Gel werden mit Hilfe einer "semi-dry"-Blottingapparatur auf Nitrozellulose übertragen. Nach Absättigung der freien Bindungsstellen der Membran mit resuspendierter Trockenmagermilch werden die Membranen mit den jeweiligen vorverdünnten anti-Peptid-Antikörper alleine oder in unterschiedlicher Kombination inkubiert. Nach intensivem Waschen der Membranen zur Entfernung aller unspezifisch gebundener Antikörper werden die Membranen mit alkalischer Phosphatase-markierten anti-Kaninchen-Antikörpern in einer vorher ermittelten Konzentration inkubiert. Die spezifischen sekundären Antikörper, die nach dem Waschen auf der Membran verbleiben, werden nach Zugabe von Substrat sichtbar gemacht. Dabei zeigte sich, das in allen Proben mit einzelnen Antikörpern oder Antikörpergemischen Elastase detektiert werden kann, jedoch nicht jeder Antikörper alle Isoformen detektiert.

### Ausführungsbeispiel 6 - Bestimmung der Pankreas-Elastase in Stuhl - und Serum unter Verwendung der erfindungsgemäßen Antikörper.

Die Elastase in Serum, Plasma oder Stuhl wird mit einem Festphasen-ELISA, der auf der Sandwichtechnik basiert, bestimmt. Dazu werden einzelne oder ein entsprechendes Gemisch mehrerer der erfindungsgemäßen Antikörper in einem Karbonat/Bikarbonat-Puffergemisch, pH 9,6 gelöst und in die Wells einer Mikrotiterplatte gegeben. Nach Inkubation bei 4 °C werden die nicht gebundenen Antikörper durch Waschen mit PBS entfernt. Die noch freien Bindungsstellen des Trägermaterials werden durch einen Ethanolamin/Tween 20-PBS-Puffer geblockt. Das Blocken findet bei Raumtemperatur über 90 Minuten statt. Nach dem Waschen werden die in PBS verdünnten Serum- bzw. Stuhlproben in die Wells pipettiert. Die 60minütiger Inkubation bei Raumtemperatur wird durch Waschen beendet. Als Detektionsantikörper werden einzelne oder ein entsprechendes Gemisch aus mehreren erfindungsgemäßen Antikörpern verwendet, die mit Biotin konjugiert wurden. Nach einer Inkubation von 30 Minuten und dem Waschprozeß wird der Biotin-markierte Antikörper mit Peroxidase-konjugiertem Streptavidin nachgewiesen. Durch den letzten Waschschritt erfolgt die Beseitigung des nicht gebundenen Streptavidins. Anschließend wird mit TMB als Substrat die Peroxidasekonzentration bestimmt. Nach Zugabe von HCl zur Beendigung der Enzymreaktion wird die Änderung der optischen Dichte gemessen. Die Intensität der Farbreaktion ist proportional der Elastase-Konzentration in der Probe.

## Patentansprüche

1. Diagnostisches Verfahren zur Erkennung einer Pankreasfunktionsstörung, **dadurch gekennzeichnet, daß** in Serum, Se- oder Exkreten eines Patienten der Gesamtgehalt der pankreatischen Elastasen 1, 2 und 3 (Isoenzyme) bestimmt wird.

2. Verfahren nach Anspruch 1, **dadurch gekennzeichnet, daß** die Bestimmung mittels immunchemischer Systeme unter Nutzung mono- oder polyklonaler Antikörper erfolgt, die die pankreatischen Elastasen 1, 2 und 3 (Isoenzyme), mit Ausnahme des Elastase-Fragmentes mit der Aminosäuresequenz Thr-Met-Val-Ala-Gly-Gly-Asp-Ile-Arg, einzeln spezifisch oder kreuzreaktiv erkennen.

3. Verfahren nach Anspruch 2, **dadurch gekennzeichnet, daß** die eingesetzten Antikörper mittels Antigenen gewonnen werden, die die kompletten pankreatischen Elastasen 1, 2 und 3 (Isoenzyme) oder Untereinheiten davon darstellen, wobei die Aminosäuresequenz Thr-Met-Val-Ala-Gly-Gly-Asp-Ile-Arg als Elastase-Fragment oder eine immunologisch wirksame Teilsequenz davon nicht verwendet wird.

4. Verfahren nach Anspruch 3, **dadurch gekennzeichnet, daß** als Antigene vorzugsweise folgende synthetische Peptide verwendet werden, die nach Immunisierung von Tieren Antikörper induzieren, die mehrere Elastasen erkennen, wobei sie die Aminosäuresequenz Thr-Met-Val-Ala-Gly-Gly-Asp-Ile-Arg nicht betreffen:
NH2-A-V-K-E-G-P-E-Q-V-I-P-I-N-COOH
NH2-Y-T-N-G-P-L-P-D-K-L-Q-Q-A-R-COOH
NH2-R-S-G-C-N-G-D-S-G-G-P-L-N-COOH
NH2-G-P-L-N-C-P-T-E-D-G-G-W-Q-COOH
NH2-G-T-E-A-G-R-N-S-W-P-S-Q-I-COOH
NH2-H-N-L-S-Q-N-D-G-T-E-Q-Y-V-COOH
NH2-W-G-K-T-K-T-N-G-Q-L-A-COOH
NH2-V-S-S-R-G-C-N-V-S-R-K-P-T-COOH
NH2-G-G-E-E-A-R-P-N-S-W-P-W-Q-COOH
NH2-S-S-S-R-T-Y-R-V-G-L-G-R-H-N-COOH
NH2-K-D-W-N-S-N-Q-I-S-K-G-N-D-COOH
NH2-G-P-L-N-C-Q-A-S-D-G-R-W-COOH
NH2-G-A-L-P-D-D-L-K-Q-G-R-L-COOH
NH2-S-L-Q-Y-E-K-S-G-S-F-Y-COOH
NH2-F-G-C-N-T-R-R-K-P-T-V-F-T-COOH

5. Verfahren nach Ansprüchen 1 - 5 **dadurch gekennzeichnet, daß** Antikörper einzeln oder in einer Kombination in immunchemischen Nachweissystemen verwendet werden.

6. Verfahren zur Gewinnung von mono- und/oder polyklonalen Antikörpern, die spezifisch mit humanen pankreatischen Elastasen 1, 2 und 3 (Isoenzyme) in Stuhl oder Körperflüssigkeiten reagieren und durch übliche Immunisierungsverfahren induziert werden, **dadurch gekennzeichnet, daß** man die Peptide A-V-K-E-G-P-E-Q-V-I-P-IN, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-I, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-K-G-N-D, G-P-L-N-C-Q-A-S-D-G-R-W, G-A-L-P-D-D-L-K-Q-G-R-L, S-L-Q-Y-E-K-S-G-S-F-Y, F-G-C-N-T-R-R-K-P-T-V-F-T oder immunogene Teilpeptide davon als Antigen zur Immunisierung von Vertebraten, insbesondere von Kleinsäugern und Vögeln, verwendet.

7. Verfahren nach Anspruch 6, **dadurch gekennzeichnet, daß** man die freien Peptide vor der Immunisierung an geeignete Trägersubstanzen, vorzugsweise Hämocyanin oder Albumin koppelt.

8. Verfahren nach Anspruch 6 und 7, **dadurch gekennzeichnet, daß** polyklonale Antikörper unter Einsatz von Hühnern als Versuchstieren produziert werden.

9. Polyklonale Antikörper, sofern sie nach Anspruch 6 bis 8 hergestellt wurden.

10. Monoklonale Antikörper, sofern sie nach Anspruch 6 und 7 hergestellt wurden.

11. Reinigungs- und Detektionssysteme für die Isoenzyme der humanen pankreatischen Elastasen 1, 2 und 3, **dadurch gekennzeichnet, daß** sie zumindest einen der erfindungsgemäßen Antikörper gemäß den Ansprüchen 9 und 10 enthalten.

12. Immunologische Testkits enthaltend einen oder mehrere Antikörper nach den Ansprüchen 9 und 10 zur Diagnose und Verlaufskontrolle von Erkrankungen der Bauchspeicheldrüse unter Verwendung von Stuhl oder Körperflüssigkeiten.

13. Immunologische Testkits nach Anspruch 12, **dadurch gekennzeichnet, daß** 2 unterschiedliche Antikörper verwendet werden (Sandwich-ELISA).

14. Reinigungs- und Detektionssysteme für Antikörper, die nach einem der Ansprüche 6 - 8 hergestellt wurden, **dadurch gekennzeichnet, daß** sie eines oder mehrere der Peptide A-V-K-E-G-P-E-Q-V-I-P-I-N, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-I, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-KG-N-D, G-P-L-N-C-Q-A-S-D-G-R-W, G-A-L-P-D-D-L-K-Q-G-R-L, S-L-Q-Y-E-K-S-G-S-F-Y, F-G-C-N-T-R-R-K-P-T-V-F-T oder immunogene Teilpeptide davon enthalten.

## Claims

1. Diagnostic procedure for identification of a disorder of the pancreas by determining the overall content of the pancreatic elastases 1, 2, and 3 (iso-enzymes) in the serum, secretions or excretions of a patient.

2. Procedure according to claim 1, **characterised by** the fact that identification is by means of immuno-chemical systems using monclonal or polyclonal antibodies that can singly specifically or cross-reactively recognise the pancreatic elastases 1, 2, and 3 (iso-enzymes) except of the elastase fragment with the amino-acid sequence Thr-Met-Val-Ala-Gly-Gly-Asp-Ile-Arg.

3. Procedure according to claim 2, **characterised by** the fact that the antibodies used are obtained by means of antigenes consisting of the complete pancreatic elastases 1, 2 and 3 or their sub-units, wherein the amino-acid sequence Thr-Met-Val-Ala-Gly-Gly-Asp-Ile-Arg or an immunologically effective partial sequence of thereof is not used as an elastase fragment.

4. Procedure according to claim 3, **characterised by** the fact that the following synthetic peptides are primarily used as antigenes which after the immunisation of animals induce antibodies which recognise several elastases but do not concern the amino-acid sequence Thr-Met-Val-Ala-Gly-Gly-Asp-lle-Arg:
NH2-A-V-K-E-G-P-E-Q-V-I-P-I-N-COOH
NH2-Y-T-N-G-P-L-P-D-K-L-Q-Q-A-R-COOH
NH2-R-S-G-C-N-G-D-S-G-G-P-L-N-COOH
NH2-G-P-L-N-C-P-T-E-D-G-G-W-Q-COOH
NH2-G-T-E-A-G-R-N-S-W-P-S-Q-I-COOH
NH2-H-N-L-S-Q-N-D-G-T-E-Q-Y-V-COOH
NH2-W-G-K-T-K-T-N-G-Q-L-A-COOH
NH2-V-S-S-R-G-C-N-V-S-R-K-P-T-COOH
NH2-G-G-E-E-A-R-P-N-S-W-P-W-Q-COOH
NH2-S-S-S-R-T-Y-R-V-G-L-G-R-H-N-COOH
NH2-K-D-W-N-S-N-Q-I-S-K-G-N-D-COOH
NH2-G-P-L-N-C-Q-A-S-D-G-R-W-COOH
NH2-G-A-L-P-D-D-L-K-Q-G-R-L-COOH
NH2-S-L-Q-Y-E-K-S-G-S-F-Y-COOH
NH2-F-G-C-N-T-R-R-K-P-T-V-F-T-COOH

5. Procedure according to claims 1 - 5, **characterised by** the fact that antibodies are used singly or in a combination in an immuno-chemical identification system.

6. Procedure for obtaining monoclonal and/or polyclonal antibodies that react specifically with human pancreatic elastases 1, 2 and 3 (iso-enzymes) in stool or body fluids and are induced by the usual immunisation procedures, **characterised by** the fact that the peptides A-V-K-E-G-P-E-Q-V-I-P-I-N, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-I, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-K-G-N-D, G-P-L-N-C-Q-A-S-D-G-R-W, G-A-L-P-D-D-L-K-Q-G-R-L, S-L-Q-Y-E-K-S-G-S-F-Y, F-G-C-N-T-R-R-K-P-T-V-F-T or immunogenic partial peptides of thereof are used as antigen for the immunisation of vertrebrates, especially of small mammals and birds.

7. Procedure according to claim 6, **characterised by** the fact that before immunisation the free peptides are coupled with suitable carrier substances, primarily haemoczanine or albumin.

8. Procedure according to claims 6 and 7, **characterised by** the fact that polyclonal antibodies are produced using chickens as experimental animals.

9. Polyclonal antibodies, insofar as they were produced according to claims 6 to 8.

10. Monoclonal antibodies, insofar as they were produced according to claims 6 and 7.

11. Purification and detection systems for the iso-enzymes of the human elastases 1, 2 and 3, **characterised by** the fact that they contain at least one of the invention antibodies according to claims 9 and 10.

12. Immunological test kits for the diagnosis and progress check of diseases of the pancreas using stool or body fluids containing one or more of the antibodies used in claims 9 and 10.

13. Immunological test kits according to claim 12, **characterised by** the fact that 2 different antibodies are used (Sandwich-ELISA).

14. Immunological test kits for the diagnosis and progress check of diseases of the pancreas and of mucoviscidosis using stool or body fluids.

15. Cleaning and detection systems for antibodies being produced according to one of the claims 6-8, **characterised by** the fact that they include one or more of the peptides A-V-K-E-G-P-E-Q-V-I-P-I-N, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-I, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-K-G-N-D, G-P-L-N-C-Q-A-S-D-G-R-W, G-A-L-P-D-D-L-K-Q-G-R-L, S-L-Q-Y-E-K-S-G-S-F-Y, F-G-C-N-T-R-R-K-P-T-V-F-T or immunologically effective partial peptides of thereof.

## Revendications

1. Procédé diagnostique de détection d'un trouble fonctionnel du pancréas **se caractérisant par le fait que** la teneur totale en élastases pancréatiques 1, 2 et 3 (isoenzymes) est déterminée dans le sérum, les sécrétions et les excréments d'un patient.

2. Procédé selon la revendication 1 **se caractérisant par le fait que** la détermination se fait au moyen de systèmes immuno-chimiques en utilisant des anticorps monoclonaux ou polyclonaux qui détectent spécifiquement ou en réaction croisée les élastases pancréatiques 1, 2 et 3 (isoenzymes) à l'exception du fragment d'élastase avec la séquence d'acide aminé Thr-Met-Val-Ala-Gly-Gly-Asp-Ile-Arg.

3. Procédé selon la revendication 2 **se caractérisant par le fait que** les anticorps employés ont été extraits au moyen d'antigènes et représentent les élastases pancréatiques 1, 2 et 3 (isoenzymes) complètes ou des sous-unités, la séquence d'acide aminé Thr-Met-Val-Ala-Gly-Gly-Asp-Ile-Arg n'étant pas utilisée en tant que fragment d'élastase ou une partie de cette séquence à action immunologique.

4. Procédé selon la revendication 3 **se caractérisant par le fait que** les peptides synthétiques suivants sont utilisés de préférence en tant qu'antigènes, peptides qui induisent des anticorps après immunisation d'animaux, qui détectent plusieurs élastases, ne concernant pas la séquence d'acide aminé Thr-Met-Val-Ala-Gly-Gly-Asp-Ile-Arg :
NH2-A-V-K-E-G-P-E-Q-V-I-P-I-N-COOH
NH2-Y-T-N-G-P-L-P-D-K-L-Q-Q-A-R-COOH
NH2-R-S-G-C-N-G-D-S-G-G-P-L-N-COOH
NH2-G-P-L-N-C-P-T-E-D-G-G-W-Q-COOH
NH2-G-T-E-A-G-R-N-S-W-P-S-Q-I-COOH
NH2-H-N-L-S-Q-N-D-G-T-E-Q-Y-V-COOH
NH2-W-G-K-T-K-T-N-G-Q-L-A-COOH
NH2-V-S-S-R-G-C-N-V-S-R-K-P-T-COOH
NH2-G-G-E-E-A-R-P-N-S-W-P-W-Q-COOH
NH2-S-S-S-R-T-Y-R-V-G-L-G-R-H-N-COOH
NH2-K-D-W-N-S-N-Q-1-S-K-G-N-D-COOH
NH2-G-P-L-N-C-Q-A-S-D-G-R-W-COOH
NH2-G-A-L-P-D-D-L-K-Q-G-R-L-COOH
NH2-S-L-Q-Y-E-K-S-G-S-F-Y-COOH
NH2-F-G-C-N-T-R-R-K-P-T-V-F-T-COOH

5. Procédé selon les revendications 1 - 5 **se caractérisant par le fait que** des anticorps sont employés individuellement ou en combinaison avec des systèmes de détection immnuno-chimique.

6. Procédé d'extraction d'anticorps mono- et/ou polyclonaux qui réagissent de façon spécifique avec des élastases pancréatiques humaines 1, 2 et 3 (isoenzymes) dans la selle ou des liquides corporels et qui sont induits par des procédés d'immunisation usuelles, **se caractérisant par le fait que** l'on utilise les peptides A-V-K-E-G-P-E-Q-V-I-P-I-N, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-I, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-K-G-N-D, G-P-L-N-C-Q-A-S-D-G-R-W, G-A-L-P-D-D-L-K-Q-G-R-L, S-L-Q-Y-E-K-S-G-S-F-Y, F-G-C-N-T-R-R-K-P-T-V-F-T ou des parties de ces peptides immunogènes en tant qu'antigène pour l'immunisation de vertébrés, en particulier de petits mammifères et d'oiseaux.

7. Procédé selon la revendication 6, **se caractérisant par le fait que** l'on couple les peptides libres avant l'immunisation à des substances de support appropriées, de préférence de l'hémocyanine ou de l'albumine.

8. Procédé selon les revendications 6 et 7, **se caractérisant par le fait que** la production d'anticorps polyclonaux se fera en employant des poules comme cobayes.

9. Anticorps polyclonaux, dans la mesure où ils ont été fabriqués selon les revendications 6 à 8.

10. Anticorps monoclonaux, dans la mesure où ils ont été fabriqués selon les revendications 6 et 7.

11. Systèmes de nettoyage et de détection pour les isoenzymes des élastases pancréatiques humaines 1, 2 et 3, **se caractérisant par le fait qu'**ils contiennent au moins un des anticorps conforme à l'invention conformément aux revendications 9 et 10.

12. Kits de test immunologiques contenant un ou plusieurs anticorps selon les revendications 9 et 10 pour le diagnostic et le contrôle de déroulement de maladies du pancréas en utilisant des selles ou des liquides corporels.

13. Kits de test immunologique selon la revendication 12, **se caractérisant par le fait que** 2 anticorps différents ont été utilisés (méthode ELISA sandwich).

14. Systèmes de nettoyage et de détection pour des anticorps qui ont été fabriqués selon l'une des revendications 6 - 8, **se caractérisant par le fait qu'**ils contienent un ou plusieurs des peptides A-V-K-E-G-P-E-Q-V-I-P-I-N, Y-T-N-G-P-L-P-D-K-L-Q-Q-A-R, R-S-G-C-N-G-D-S-G-G-P-L-N, G-P-L-N-C-P-T-E-D-G-G-W-Q, G-T-E-A-G-R-N-S-W-P-S-Q-1, H-N-L-S-Q-N-D-G-T-E-Q-Y-V, W-G-K-T-K-T-N-G-Q-L-A, V-S-S-R-G-C-N-V-S-R-K-P-T, G-G-E-E-A-R-P-N-S-W-P-W-Q, S-S-S-R-T-Y-R-V-G-L-G-R-H-N, K-D-W-N-S-N-Q-I-S-K-G-N-D, G-P-L-N-C-Q-A-S-D-G-R-W, G-A-L-P-D-D-L-K-Q-G-R-L, S-L-Q-Y-E-K-S-G-S-F-Y, F-G-C-N-T-R-R-K-P-T-V-F-T ou des parties de ces peptides immunogènes.
